# EUROPEAN PATENT APPLICATION

(11) **EP 4 439 585 A1**
(43) Date of publication of application: **02.10.2024**
(21) Application number: 23164513.6
(22) Date of filing: 28.03.2023
(51) Int. Cl.: G16H 50/20, G16B 40/20, G16H 50/30

(54) **SYSTEM AND METHOD TO IMPROVE CLINICAL DECISION MAKING BASED ON GENOMIC PROFILES BY LEVERAGING 3D PROTEIN STRUCTURES TO LEARN GENOMIC LATENT REPRESENTATIONS**

(71) Applicant: Siemens Healthineers AG, 91301 Forchheim (DE)
(72) Inventor: Siebert, Matthias, 91080 Marloffstein (DE)
(74) Representative: Siemens Healthineers Patent Attorneys

(57) **Abstract**

The present invention relates to a computer-implemented method for the analysis of genomic sequence data comprising the steps: obtaining genomic sequence data, obtaining data from three-dimensional protein structures; mapping the genomic sequence data obtained on the protein structures obtained; inputting the mapped genomic sequence data into a trained graph neural network, preferably a graph convolutional network, a graph isomorphism network or a graph attention network, wherein the architecture of the graph neural network is based on the three-dimensional protein structure and wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition; and deriving a prognostic and/or predictive conclusion output with respect to said disease or medical condition. Further envisaged are a corresponding data processing device and computer program.

## Description

The present invention relates to a computer-implemented method for the analysis of genomic sequence data comprising the steps: obtaining genomic sequence data; obtaining data from three-dimensional protein structures; mapping the genomic sequence data obtained on the protein structures obtained; inputting the mapped genomic sequence data into a trained graph neural network, preferably a graph convolutional network, a graph isomorphism network or a graph attention network, wherein the architecture of the graph neural network is based on the three-dimensional protein structure and wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition; and deriving a prognostic and/or predictive conclusion output with respect to said disease or medical condition. Further envisaged are a corresponding data processing device and computer program.

The genetic makeup of cells contributes to, or even determines, disease etiology, as is, for example, the case for cancer, a genetic disease that develops in a multistep process by progressively acquiring somatic mutations in oncogenes and tumor suppressor genes of a tissue that transform a normal into a malignant cell. Thus, genotyping technologies, such as next-generation sequencing, are increasingly being employed to support clinical decision-making. However, the size of the genomic territory in combination with small patient cohorts as well as inherent genetic heterogeneity, i.e., different genotypes resulting in the same phenotype, render it very difficult to suitably train clinical decision support systems that consider the fully available genomic information.

To cope with this problem, a set of significant mutations can be preselected, e.g., by independently testing single gene mutations for statistically significant association with a certain phenotype, or a summary statistic is used to aggregate mutations that occur in the same gene, e.g., by encoding whether a gene harbors a non-synonymous mutation as a binary feature. Subsequently, simplified models are trained based on thus selected mutations or composite features as described in Elmarakeby et al., 2021, Nature, Vol. 598, 348-352 or Yousefi et al., 2017, Scientific Reports, 7, 11707.

Both approaches may reduce biological noise, e.g., by ignoring mutations that do not appear to be relevant to the prediction task, at least when assessed independently of other mutations, or by aggregating evidence from sparse mutation data on a higher concept level, e.g., on the level of genes or biological pathways. However, positions that are distant in the gene sequence may encode amino acids that are closely located in the three-dimensional protein structure, e.g., forming an active site or a protein-protein interface. Hence, in order to effectively assess the collective impact of gene mutations on the phenotype, it is necessary to also consider the resulting epistatic patterns of mutual exclusivity and co-occurrence of mutations. There is thus a need for a method and system which leverages the fully available genomic information to improve clinical decision-making. Further, the method and system is required to incorporate prior knowledge on interactions between specific genomic positions of a gene to enable more robust training from high-dimensional genomic data and the explicit modeling of genetic interactions should enable improved model interpretability which allows clinical adoption.

The present invention addresses these needs and provides in a first aspect a computer-implemented method for the analysis of genomic sequence data comprising the steps: (a) obtaining genomic sequence data; (b) obtaining data from three-dimensional protein structures; (c) mapping the genomic sequence data obtained in step (a) on the protein structures obtained in step (b); (d) inputting the mapped genomic sequence data of step (c) into a trained graph neural network, preferably a graph convolutional network, a graph isomorphism network or a graph attention network
- wherein the architecture of the graph neural network is based on the three-dimensional protein structures,
- wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition; and
(e) deriving a diagnostic, prognostic and/or predictive conclusion output with respect to said disease or medical condition.

This method generally improves clinical decision-making of patients from genomic data. It can advantageously be applied to a multitude of clinical decision-making tasks that depend on or benefit from the consideration of genomic data such as the prediction of disease subtypes, the progression of a disease or the response rate to a treatment. By further integrating secondary data concerning the disease phenotype and personal particulars of a patient, a robust diagnostic, prognostic and predictive tool was developed. In addition, it can advantageously be applied to small patient cohorts as compared to a method that does not incorporate prior knowledge on interactions between specific genomic positions of a gene. Moreover, the graph architecture enables the identification of subgraphs, i.e., protein substructures, important for conclusion outputs.

In a preferred embodiment of the present invention, the output is provided in the form of a metric score within a clinical decision scale.

In another preferred embodiment the data from three-dimensional protein structures include data for protein-protein interactions or protein-protein complexes.

In a further preferred embodiment, the data from three-dimensional protein structures, protein-protein interactions or complexes are derived from a protein structure database or protein structure prediction database. It is particularly preferred that the data are derived from PDB or AlphaFold DB.

In another preferred embodiment the mapping of the genomic sequence data to the protein structure data incorporates information on a predicted impact of mutations on the protein sequence, structure and function. It is particularly preferred that said information on the impact is provided by a variant effect predictor.

The present invention relates, in yet another preferred embodiment, to the additional training of the graph neural network by inputting data of a cohort of subjects affected by a disease or medical condition concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome.

It is further preferred that a target subject's data concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome, is obtained and inputted into the trained graph neural network.

In another preferred embodiment the graph neural network comprises nodes and edges, wherein said nodes correspond to the Calpha atom of the amino acid glycine or the Cbeta atom of amino acids other than glycine.

According to a further preferred embodiment, the edges of the graph neural network link the Cbeta or Calpha atoms of the amino acids that have a Euclidian distance of below a predetermined threshold, preferably a threshold of 6 to 12 Angström, more preferably of 7 Angström.

In an additional preferred embodiment, the edges of the graph neural network comprise weights which correspond to a function of said Euclidian distance.

The present invention further envisages that the genomic sequence data is obtained from a panel sequencing, a whole-exome sequencing or a somatic genomic sequencing, wherein optionally a preselection of genomic sequence data with respect to available protein structures, known mutation locations and/or a disease or medical condition of interest is performed.

It is further preferred that the diagnostic and/or therapeutic conclusion output comprises a disease subtype classification and/or a prognostic trend assessment and/or a treatment response prediction.

In a further aspect the present invention relates to a data processing device or system for the analysis of genomic sequence data, comprising means for carrying out the method according to the present invention.

In an additional aspect the present invention relates to a computer program comprising instructions which, when the program is executed by a computer, cause the computer to carry out the computer-implemented method according to the present invention.

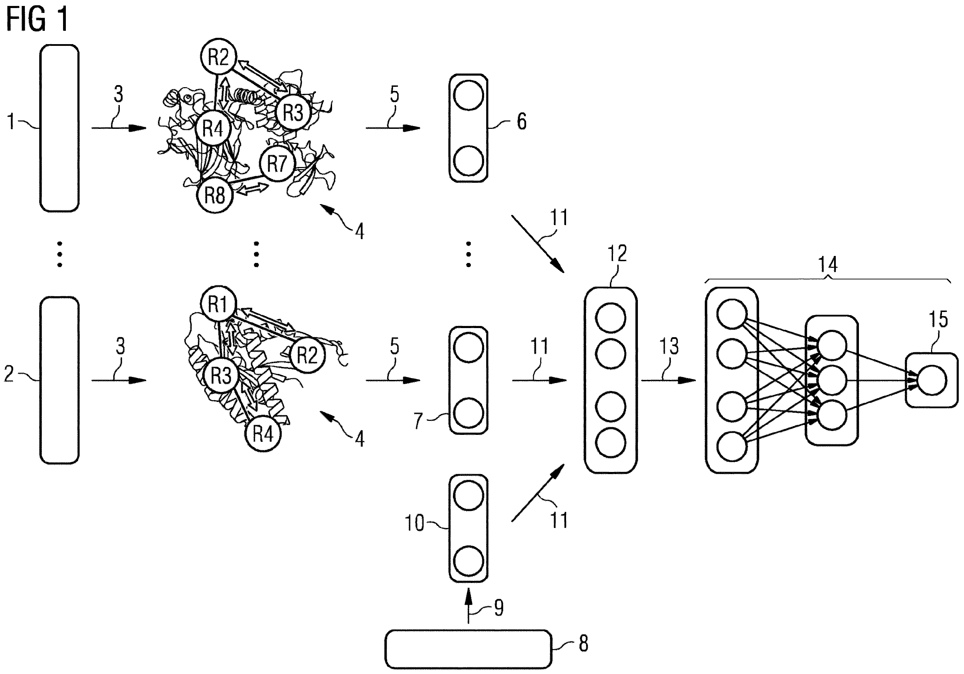

FIG. 1 shows a schematic illustration of an embodiment of the invention. On the left side genomic sequence data including information on mutations for gene 1 (1) up to gene N (2) is shown. The sequence encodes a protein which is mapped (3) on corresponding protein structures to generate a graph neural network architecture (4). The nodes (R1, R2, R3, R4, R7, R8) show amino acid residues, the edges between theses nodes depict the pairwise Euclidean distance between the Cbeta atoms of amino acids below a certain threshold. The network uses message passing which allows to consider edge weights as a function of Euclidian distance. The network is consulted for an embedding output, by aggregating the feature vectors of graph nodes after message passing, e.g., using pooling (5) to obtain embeddings for gene 1 (6) up to gene N (7).

Additional information, e.g., on disease phenotypes, age, race or sex implications, molecular characteristics on the level of the transcriptome, metabolome, etc. (8) may be inputted (9) in an encoded form (10). The encoding of additional information (10) is further concatenated with the embeddings of all genes (6, 7) to yield layer (12) which is inputted (13) into a multilayer perceptron (MLP)(14) producing a prediction label as outcome (15).

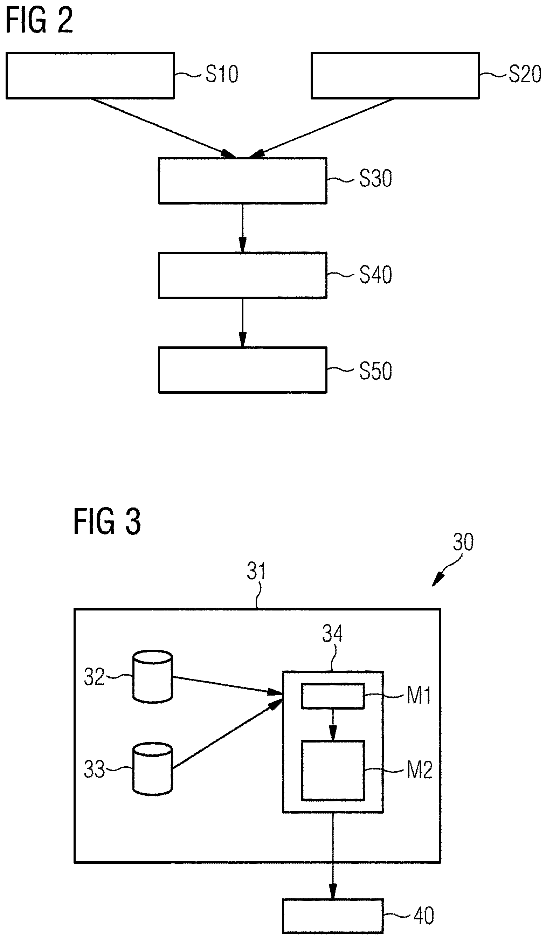

FIG. 2 shows a schematic illustration of method steps according to an embodiment of the present invention. In step S10 genomic sequence data are obtained. In step S20 data from three-dimensional protein structures are obtained. Step S30 encompasses the mapping of the genomic sequence data previously obtained in step S10 on the protein structures previously obtained in step S20. Subsequently (S40) the mapped genomic sequence data of S30 is inputted into a trained graph neural network. Finally, in step S50, a diagnostic, prognostic and/or predictive conclusion output is derived.

FIG. 3 depicts a data processing system according to an embodiment of the present invention. The system (30) comprises a device (31) comprising a unit for obtaining and storing genomic sequence data (32), a unit for obtaining and storing three-dimensional protein structures (33), a processor (34) comprising a module M1 which is designed to map the genomic sequence data on the protein structures obtained, and a module M2, which comprises a trained graph convolutional network, into which the mapped data of M1 is inputted. The processor provides a diagnostic, prognostic and/or predictive conclusion output (40).

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms " (i) ", "(ii)", "(iii)" or " (a) ", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a computer-implemented method for the analysis of genomic sequence data comprising the steps: (a) obtaining genomic sequence data; (b) obtaining data from three-dimensional protein structures; (c) mapping the genomic sequence data obtained in step (a) on the protein structures obtained in step (b); (d) inputting the mapped genomic sequence data of step (c) into a trained graph neural network, preferably a graph convolutional network, a graph isomorphism network or a graph attention network
- wherein the architecture of the graph neural network is based on the three-dimensional protein structures,
- wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition; and
(e) deriving a diagnostic, prognostic and/or predictive conclusion output with respect to said disease or medical condition.

The term "genomic sequence data" as used herein refers to sequence data obtained by any technique suitable to provide sequence data of an organism's genome. The genomic sequence may comprise any genomic sequence segment which is considered of interest for the determination of the presence of a mutation. It may preferably include coding sequences, but also non-coding sections may be included such as regulatory sequences, regulatory active intron sequences etc., which can be introduced into the GNN as additional vector. In a preferred embodiment the genomic sequence may include primarily exonic sequences, or exonic sequences only. The genomic sequence data may further be limited to certain chromosomes, chromosomal regions, gene clusters, gene families, or genes including or excluding regulatory elements in the vicinity. It is particularly preferred that the genomic sequence data is obtained from a panel sequencing, a whole-exome sequencing or a somatic genomic sequencing.

The term "obtain" or "obtained" as used herein means that data may be received, e.g. from a database, or otherwise be supplied, provided or allocated. The term includes all activities which lead to the presentation and, optionally, storage of the data in a form suitable for the method according to the present invention.

The genomic sequence data may be obtained with any suitable technology, preferably in a high-throughput approach. This typically includes next-generation sequence (NGS) or secondgeneration sequencing techniques. Such approaches comprise any sequencing method that determines the nucleotide sequence of either individual nucleic acid molecules or expanded clones for individual nucleic acid molecules in a highly parallel fashion. The sequencing may be performed according to any suitable massive parallel approach. Typical platforms include Roche 454, GS FLX Titanium, Illumina, Life Technologies Ion Proton, Oxford Nanopore Technologies, Solexa, Solid or Helicos Biosciences Heliscope systems.

Obtaining genomic sequence data means that any suitable massively parallel sequencing approach may be performed, or that the data is derived from an information or sequence repository or database.

In certain embodiments, the sequencing may include the preparation of nucleic acids, the sequencing, as well as subsequent imaging and initial data analysis steps.

Preparation steps may, for example, include randomly breaking genomic nucleic acids into smaller sizes and generating sequencing templates such as fragment templates. Spatially separated templates can, for example, be attached or immobilized at solid surfaces which allows for multiple sequencing reactions to be performed simultaneously. In typical examples, a library of nucleic acid fragments is generated and adaptors containing universal priming sites are ligated to the end of the fragments. Subsequently, the fragments are denatured into single strands and captured by beads. After amplification and a possible enrichment, e.g., as defined in more details herein below, a huge number of templates may be attached or immobilized in a polyacrylamide gel or be chemically crosslinked to an amino-coated glass surface, or be deposited on individual titer plates. Alternatively, solid phase amplification may be employed. In this approach forward and reverse primers are typically attached to a solid support. The surface density of amplified fragments is defined by the ratio of the primers to the template on the support. This method may produce millions of spatially separated template clusters which can be hybridized to universal sequencing primers for massively parallel sequencing reactions. Further suitable options include multiple displacement amplification methods.

Suitable sequencing methods include, but are not limited to, cyclic reversible termination (CRT) or sequencing by synthesis (SBS) by Illumina, sequencing by ligation (SBL), single-molecule addition (pyrosequencing) or real-time sequencing. Exemplary platforms using CRT methods are Illumina/Solexa and HelicoScope. Exemplary SBL platforms include the Life/APG/SOLiD support oligonucleotide ligation detection. An exemplary pyrosequencing platform is Roche/454. Exemplary real-time sequencing platforms include the Pacific Biosciences platform and the Life/Visi-Gen platform. Other sequencing methods to obtain massively parallel nucleic acid sequence data include nanopore sequencing, sequencing by hybridization, nano-transistor array-based sequencing, scanning tunneling microscopy (STM) based sequencing, or nanowire-molecule sensor-based sequencing. Further details with respect to the sequencing approach would be known to the skilled person or can be derived from suitable literature sources such as Goodwin et al., Nature Reviews Genetics, 2016, 17, 333-351, or van Dijk et al., Trends in Genetics, 2014, 9, 418-426.

Correspondingly obtained data are provided in the form of sequencing reads which may be single-end or paired-end reads. Obtaining such sequencing data may further include the addition of assessment steps or data analysis steps.

Furthermore, the presently described methodology may be used with any suitable sequencing read length. It is preferred to make use of sequencing reads of a length of about 50 to about 2000, or about 75 to about 1500 nucleotides, e.g., 50, 60, 70, 80, 90, 100, 110, 120, 130, 140, 150, 160, 170, 180, 190, 200, 500, 700, 1000, 1500, 2000 or more nucleotides or any value in between the mentioned values.

The genomic sequence data may further be assembled into contigs or suitable subsections thereof, e.g., on the basis of chromosomes, chromosome portions etc., and/or be aligned to a reference sequence, e.g., an annotated genomic sequence to detect mutations or aberrations. It is preferred that the alignment and comparison step further include a reference searching step of literature knowledge concerning identified mutations or aberrations. The method, in particular, envisages to obtain a target subject's or patient's genomic sequence data. The data may be obtained and processed according to procedures as mentioned above. In certain embodiments, obtaining genomic sequence data from a subject or patient may additionally include a preparation step for nucleic acids, which comprises a hybrid-capture based nucleic acid enrichment for genomic regions of interest, e.g., previously designated sequences, genomic regions or genes of interest such as exonic sequences, sequences associated with a certain disease or condition, mutational hot spot sequences etc. The term "hybrid-capture based nucleic acid enrichment" as used herein, means that firstly a library of nucleic acids is provided, which is subsequently contacted with hybrid capture probes, either being in solution or being immobilized on a substrate, which comprises a plurality of baits, e.g. oligonucleotide baits complementary to a gene or genomic region of interest to form a hybridization mixture; and subsequently separating a plurality of bait/nucleic acid hybrids from the mixture, e.g. by binding to an entity allowing for separation. This enriched mixture may subsequently be purified or further processed. The identity, amount, concentration, length, form etc. of the baits may be adjusted in accordance with the intended hybridization result. Thereby, a focusing on a gene or region of interest may be achieved, since only those fragments or nucleic acids are capable of hybridizing which show complementarity to the bait sequence. The present invention envisages further variations and future developments of the above-mentioned approach. Further details would be known to the skilled person or can be derived from suitable literature sources such as Mertens et al., 2011, Brief Funct Genomics, 10(6), 374-386; Frampton et al., 2013, Nature Biotechnology,31(11), 1023-1031; Gnirke et al., 2009, Nature Biotechnology,27(2), 182-189 or from Teer et al, 2010, Genome Res, 20(10), 1420-1431.

According to the present invention the genomic sequence data may be obtained from a sample derived from a subject or group of subjects. The sample may be derived from any patient or subject afflicted by a disease or condition or suspected to be afflicted by a disease or condition, or from any other subject, e.g., for control or reference purposes. In certain embodiments, the sample is a tumor sample, i.e., the nucleic acids may be extracted from a tumor of a subject, in other embodiments the sample may be a tissue, urine, blood, semen, liquor, plasma, serum or other sample. Particularly preferred are liquid biopsy samples derived from blood/urine or other body fluids. Also envisaged is to make use of previously deposited samples, e.g., samples derived from the umbilical cord.

Further, the genomic sequence data may comprise or be associated to genetic information known to be linked with a specific disease or medical condition. For example, if a disease is known to be associated with the expression or nonexpression of a gene or if certain mutations in a gene or group of genes have been shown to have an influence on the presence or prognosis of a disease the genomic sequence data may be restricted to corresponding sections of the genome. In addition, the present invention envisages a preselection of genomic sequence data with respect to available protein structures, known mutation locations and/or a disease or medical condition of interest.

The computer-implemented method further comprises a step of obtaining data from three-dimensional protein structures, i.e., a step of data collection of three-dimensional protein structures. The term "three-dimensional protein structure" as used herein refers to a set of data which define the coordinates of each atom in a protein. The coordinates can be experimentally determined using X-ray crystallography or NMR. These techniques allow to elucidate a protein's secondary structure, i.e., the arrangement of a polypeptide into an ordered substructure, e.g., an alpha helix or a beta sheet, or a protein's tertiary structure, i.e., a structure wherein the entire polypeptide has folded into a three-dimensional form. It is preferred that the data collected within the herein described computer-implemented method reflect the tertiary structure of a protein. Data concerning the three-dimensional structure may be derived from any suitable database. It is preferred to use the Protein Data Bank (PDB) data, or any suitable modified or extended version thereof. The PDB comprises for example information on unit cell parameters, space groups and Z values. Further coordinate system information, e.g., on orthogonal coordinate system transformations is provided. Typically, the coordinates are provided as coordinates of a Cartesian coordinate system. The PDB is publicly accessible over the internet, e.g., at https://www.wwpdb.org/ (last visited on March 8, 2023). A further example of a suitable database and corresponding structure file format envisaged in the present invention is the Computed Structure Model (CSM) which can be obtained from RCSB, which can be publicly accessed at https://www.rcsb.org (last visited on March 8, 2023).

In specific embodiments, the three-dimensional structure data may alternatively be derived from protein structure prediction databases. Examples include the AlphaFold Database, which comprises three-dimensional structure information on amino acid sequences predicted by the AI system AlphaFold. The database is publicly accessible over the internet, e.g., at https://alphafold.ebi.ac.uk/ (last visited on March 8, 2023).

In specific embodiments the three-dimensional structure data may also include data on protein-protein interactions or protein-protein complexes, e.g., quaternary structures or multi-protein complexes, protein-peptide interactions, protein-small molecule interactions, active interfaces etc. These data may for example, be derived from suitable expert databases or any other suitable information repository. Examples for databases include the Protein Common Interface Database (ProtCID), which provides clusters of interfaces of full-length protein chains as means of identifying biological assemblies. The database is publicly accessible over the internet, e.g., at http://dunbrack2.fccc.edu/protcid/ (last visited on March 8, 2023). Alternatively, or additionally, information may be derived from database PrePPI, which combines predicted and experimentally determined protein-protein interactions using a Bayesian framework. The database is publicly accessible over the internet, e.g., at http://bhapp.c2b2.columbia.edu/PrePPI (last visited on March 8, 2023). Further envisaged is the use of database PepBDB, which provides information on biological peptide-protein interactions. The database is publicly accessible over the internet, e.g., at http://huanglab.phys.hust.edu.cn/pepbdb/ (last visited on March 8, 2023).

The data is obtained in any suitable format, e.g., as PDBx/mmCIF, PDB or XML.

The data may be stored locally or in a cloud system or may be derived from the database on-the-fly. The step of obtaining data may, in certain embodiments, also include an update functionality which searches for changes to three-dimensional structure data in the database. This update may, for example, be performed within predefined period, e.g., every week, 2, 3, 4 weeks, 2 months, 3, months, 4 months etc.

The data may, in specific embodiments, be modified or processed, e.g., transformed into a new format, analyzed with respect to a feature of interest, or combined in any suitable way.

The obtained genomic sequence data is subsequently mapped to the protein structures initially obtained, as described herein above. The "mapping" is performed by a comparison of the nucleotide sequence via the encoded primary amino acid sequence with the three-dimensional structure of the protein.

The mapped subject's genomic sequence data is then inputted into a trained graph neural network (GNN). The term "graph neural network (GNN)" as used herein refers to a neural network which is composed of nodes or vertexes, representing data entities, and edges between these nodes, which represent relations between the nodes. The network may further comprise information on directionality, e.g., as undirected or directed connections between nodes.

Typically, the GNN is based on a neighborhood aggregation or message passing scheme, where the representation vector of a node is computed recursively aggregating and transforming representations vector of its neighboring nodes, i.e., each node aggregates feature vectors of its neighbors to compute its new feature vector. Generally, after a certain number of iterations of aggregation, a node is represented by its transformed feature vector, which captures the structural information within the nodes' neighborhood. The representation of an entire graph can then, for example, be obtained through pooling, e.g., by summing the representation vectors of all nodes in the graph.

The GNN is accordingly to be understood as an optimizable transformation on all attributes of the graph, e.g., nodes, edges and global context which preserve graph symmetries. Typically, the GNN provides a model for each protein in the form of a separate graph, i.e., one protein structure may be represented by one graph. In case of structures of protein-protein complexes, one graph may correspond to a complex of two or more interacting proteins. In case of interacting surfaces or the like, the graphs of two proteins may be connected, e.g., via the amino acids which participate at the interaction surface or region.

The graph neural network is typically connected to an adjacency matrix. The term "adjacency matrix" is understood within the context of the present invention as a square matrix used to represent a finite graph. The elements of the matrix indicate whether pairs of nodes are adjacent or not in the graph. In addition, or alternatively, an adjacency list may be generated, wherein said list describes the connectivity of edges between nodes as tuples. The adjacency matrix preferably comprises contact maps of the Euclidian distances within a protein as described herein.

The GNN thus comprises a number of graphs, which essentially correspond to the different protein structures obtained initially, wherein the network is structured in so called GNN layers. Accordingly, the architecture of the trained GNN is based on the three-dimensional protein structure obtained.

Optionally, for edges and global-context MLPs may be trained to yield edge or global feature vectors. The layers may further be stacked. The graphs are converted into vectors generating embeddings, e.g., by pooling node-specific embeddings, i.e., for every graph an embedding is generated.

The embeddings can be pooled or concatenated and serve as input for a task-specific multilayer perceptron (MLP), e.g., a prediction MLP. Further details can be derived from Fig. 1.

In the context of the three-dimensional protein structures the nodes of a graph neural network may preferably represent Calpha and Cbeta atoms and the edges may represent a Euclidean distance between the Calpha or Cbeta atoms of a pair of amino acids below a predetermined threshold.

In preferred embodiments, the nodes of the graph neural network correspond to the Calpha atom of the proteinogenic amino acid glycine or the Cbeta atom of proteinogenic amino acids other than glycine. In the case of the proteinogenic alpha-amino acids the Calpha atom is the carbon atom to which the amino group and the carboxyl group are attached. Cbeta atoms in proteinogenic alpha-amino acids are the first atoms of the amino acids' sidechains. Since glycine has no sidechain, the Calpha atom is used instead for the definition of the nodes. Correspondingly characterized nodes provide a clear definition of the protein structure in terms of graph reflected three-dimensional positions. In certain specific embodiments, the coordinates of atoms may also be stored.

In further preferred embodiments, the edges of the graph neural network link the Cbeta atoms of the proteinogenic amino acids other than glycine and the Calpha atoms of glycine in case these Cbeta or Calpha atoms have a Euclidian distance of below a predetermined threshold. The term "Euclidian distance" as used herein means a distance between two points in Euclidean space, i.e., the length of a line segment between the two points. It is typically calculated from the Cartesian coordinates of the points using the Pythagorean theorem. Accordingly, the structure information derived from database entries as mentioned above, e.g., PDB data, can be transformed into Euclidian distances in accordance with the present invention.

In specific embodiments the threshold for Euclidian distances is 6 to 12 Angström, e.g., 6, 7, 8, 9, 10, 11, 12 Angström, or any value in between the mentioned values. It is preferred that the threshold is at 7 Angström. This threshold advantageously allows to reflect the reconstruction of the protein's structure in a sufficiently high quality.

In further specific embodiments the nodes and/or edges of the graph neural network may comprise attributes. Further, the network may optionally comprise global or master node attributes, e.g., referring to the number of nodes or the longest/shortest path between them etc. For example, the nodes may comprise an attribute as to its identity, the number of neighbors or the like. The edges may, for example, comprise attributes as to the edge identity or an edge weight. It is preferred that the edge weight corresponds to a function of the Euclidian distance between the nodes. The term "function of the Euclidian distance" refers, for example, to the inverse Euclidian distance or the modification of a threshold value for an edge weight.

The present invention envisages that the GNN may have any suitable form or architectural type. In one preferred embodiment the GNN may have the form/structure of a graph convolutional network (GCN). A GCN is a network, which shares filter parameters over all locations in the graph. Further details can be derived from suitable literature references such as Xu et al., 2019, How powerful are graph neural networks, Proceedings of ICLR, or Kipf and Welling, 2017, Semi-supervised classification with graph convolutional networks, Proceedings of ICLR.

In one preferred embodiment the GNN may have the form/structure of a graph isomorphism network (GIN). A GIN implements an aggregation scheme that represents universal functions over a node and the multiset of its neighbors. The GIN typically trains a separate multilayer perceptron (MLP) on each component of the graph, yielding GIN layers. Accordingly, for each node vector the MLP is applied and yields a node feature vector. Further details can be derived from suitable literature references such as Xu et al., 2019, How powerful are graph neural networks, Proceedings of ICLR.

In a further preferred embodiment, the GNN may have the form/structure of a graph attention network (GAT). The GAT operates on graph-structured data. Generally, the attention mechanism deals with variable sized inputs, focusing on the most relevant parts of the input to make decisions. The GAT computes hidden representations of each node in the graph by attending over its neighbors, following a self-attention strategy. Further details can be derived from suitable literature references such as Velickovic et al., 2018, Graph attention networks, Proceedings of ICLR, or Brody et al., 2021, How Attentive are Graph Attention Networks?, Proceedings of ICLR.

The trained graph neural network (GNN) is or has been trained based on genomic sequence data from a cohort of subjects affected by a disease or medical condition. Accordingly, genomic data is associated with a certain cohort or group of persons which are known to be affected by a disease or medical condition. The group may be affected by the same disease or condition, or by a family or group of similar diseases or conditions. In addition, genomic data of one or more healthy subjects is additionally inputted and is required for reference and comparison purposes.

It is preferred that said disease or medical condition is reflected by the subject's genomic sequence, e.g., in the form of a detectable mutation. Such a mutation may be an insert or a deletion or a substitution of one or more nucleotides. Such mutations may, in further preferred embodiments, lead to changes in the primary amino acid sequence of a protein.

The "disease" or "medical condition" as used herein refers to any disease or condition which is caused or contributed by a genetic aberration leading to a modification of one or more cellular proteins. For example, the disease or condition may be cancer, or a genetic disorder such as a hereditary genetic disease. Examples of diseases or medical conditions include cancer such as stomach, colon, rectal, liver, pancreatic, lung, breast, cervix uteri, corpus uteri, ovary, prostate, testis, bladder, renal, brain/CNS, head and neck, throat, Hodgkin's disease, non-Hodgkin's lymphoma, multiple myeloma, leukemia, melanoma skin cancer, non-melanoma skin cancer, acute lymphocytic leukemia, acute myelogenous leukemia, Ewing's sarcoma, small cell lung cancer, choriocarcinoma, rhabdomyosarcoma, Wilms' tumor, neuroblastoma, hairy cell leukemia, mouth/pharynx, oesophagus, larynx, kidney cancer, lymphoma, or any subtype thereof; or neurologic diseases such as Alzheimer's disease, multiple sclerosis, Amyotrophic Lateral Sclerosis (ALS), or ataxia, or cardiovascular diseases such as coronary heart diseases; or metabolic diseases such as diabetes, metabolic syndrome, iron metabolism disorders, lipid metabolism disorders, disorders of calcium metabolism etc. Further, the disease or condition may be a predisposition for a disease which is reflected by the presence of a combination of gene variants or gene modifications.

In further embodiments, specific genes may be preselected which are known for their implication in disease etiology. For example, in the context of cancer genes that are annotated in the COSMIC Cancer Gene Census and carry one or more non-synonymous mutations may be selected. Preferably, e.g., in the context of cancer, the genes encoding TP53, KRAS, KEAP1, STK11, EGFR, NF1, BRAF or STED2 may be preselected and corresponding genomic sequence data be obtained. Further specific examples of genes involved in different classes of diseases would be known to the skilled person or can be derived from suitable databases such as https://www.disgenet.org/ (last visited on March 13, 2023), which provides information on the association of genes and diseases.

The training of the GNN is further based on the mapping of the genomic sequence data obtained from the cohort of patients on the protein structures present in the graph neural network. Preferably, in an initial step all mutations are assigned to the protein sequence, e.g., in the VCF format. This step may further be influenced by additional information, e.g., on the expression of the protein, or on tissue or environmental associations. The amount and type of information which is inputted during the mapping process may be controlled and modified via additional tools such as the Variant Effect Predictor (VEP). For example, after employment of such a tool, it can be determined whether a modification of a codon sequence results in a change of an amino acid in the protein. Subsequently, a binary coding scheme may be used, which assigns a 1 to each changed amino acid and a 0 to all unchanged amino acids. This can advantageously assign a one-dimensional feature vector as input to each node corresponding to an amino acid. In addition, information about the presumed effect of the amino acid modification or change may be encoded, e.g., in accordance with a classification of the impact of mutation as defined herein below.

Preferably, the mapping of the genomic sequence data to the protein structure data incorporates information on a predicted impact of mutations on the protein sequence, structure and function. The "impact of a mutation" may, for example be classified as the absence of a mutation, the presence of a synonymous mutation, the presence of a moderate impact mutation, or the presence of a high impact mutation. A high impact mutation may, for example, be a mutation which results in a significant structural or functional modification. A moderate impact mutation may be, for example, a mutation which changes or modifies a structure or function, but has no significant effect, e.g., a functional reduction by 5, 10, 15% or a structural modification in the range of 5, 10, 15% reduction of structural identity. For example, the effect of replacing two hydrophobic amino acids such as leucine and isoleucine is considered to be a moderate impact mutation, whereas a change from leucine to arginine is considered to have a more serious effect on the function of the protein and/or its structure and is thus classified as high impact mutation. Accordingly, a two-dimensional (1-hot-encoded) feature vector is preferably envisaged. In case a mutation introduces a frame shift or a stop codon into the primary sequence, which is seen has highest impact mutation, the severity of this change may be encoded by marking the corresponding amino acid and all following amino acids in the protein sequence as modified or mutated.

The encoding may preferably be performed using a one-hot encoding, wherein the state of the mutation is reflected. The impact may in particularly preferred embodiments be determined with the help of a variant annotation and effect prediction tool. Examples of these variant effect predictor tools are the programs VEP, SIFT, PolyPhen-2 and SnpEff. Preferred is the use of VEP.

Further the training of the graph neural network comprises a step of connecting the mapped genomic sequence data and diagnostic, prognostic and/or predictive conclusions in the context of the disease or medical condition corresponding to the genomic sequence data from a cohort of subjects affected by a disease or medical condition as described above. The term "diagnostic, prognostic and/or predictive conclusions" as used herein refers to information on medical or therapeutic consequences of certain features of the genomic sequence data. For example, if a certain mutation which is found in the genomic sequence data is connected to a disease or medical condition, this connection or link may be translated into a diagnostic output, e.g., reflected by a node of the GNN and/or a corresponding edge. Corresponding information may, for example, be derived from suitable literature sources or database entries associated with the mutation. Similarly, if a certain mutation which is found in the genomic sequence data is connected to early symptoms of a disease or medical condition etc., this connection or link may be translated into a prognostic output, e.g., reflected by a node of the GNN and/or a corresponding edge. Corresponding information may, for example, be derived from suitable literature sources or database entries associated with the mutation. Also, should a certain mutation which is found in the genomic sequence data be connected to therapeutic suggestions or instructions in the context of a disease or medical condition, this connection or link may be translated into a predictive output, e.g., reflected by a node of the GNN and/or a corresponding edge. Corresponding information may, for example, be derived from suitable literature sources or database entries associated with the mutation.

The training essentially modifies the weights of the nodes and edges according to the inputted data but leaves the architecture of the graphs unchanged. For each task during the training a corresponding prediction module (MLP) is required. The GNN prediction approach, which is established during the training, is centrally based on pooling and concatenating information items. For example, the pooling typically comprises a collection of embeddings for an information item and its concatenation into a matrix. Correspondingly gathered embeddings are then aggregated, e.g., with a max operation. In particular, the present invention envisages an end-to-end training, which adjusts the trainable weights to minimize the discrepancy between prediction and actual labels depending on a loss function. The network can, for example, be adapted to the prediction task, e.g., by using a Cox loss module in a time-to-event prediction task. Accordingly, after a certain number of iterations, the training loss can be reduced. The training may further include the use of validation data which is employed to test and select the trained network. On the basis of a repeated use of the validation data a final trained network may be obtained.

The trained GNN thus reflects information on disease states being linked to genomic mutations via structural representations of estimated changes to encoded amino acid sequences. This connection advantageously allows to associate modifications in the nucleotide sequence and primary amino acid sequence with three-dimensional structural positions, thus elucidating functional correlations, which are not derivable from linear sequences such as nucleotide or primary amino acid sequences. In particular, while there are a few recurrent mutations, i.e., mutations that recur in patients with the same disease state, many mutations occur at different positions of the same gene across patients. Consequently, the number of samples carrying a specific mutation is low. However, disparate mutations on the level of nucleotide sequences can result in changes in the same amino acid or in amino acids that are close in the three-dimensional protein structure. By modeling the effect of mutations on the level of protein structures, the effective number of samples, e.g., the number of samples carrying a mutation in the active site of a protein, can thus be increased.

It is preferred that the pooling and concatenating activities are performed with message passing. The term "message passing" as used herein generally refers a set of at least two phases, a message passing phase and a readout phase, wherein the message passing phase is defined in terms of message and node update functions. During this phase hidden states at each node in the graph are updated based on the messages. During the readout phase a feature vector for the whole graph is calculated using a certain readout function. The functions are typically learned differentiable functions. Further details can be derived from suitable references such as Gilmer et al., Proceedings of the 34th International Conference on Machine Learning, Sydney, Australia, PMLR 70, 2017. In specific embodiments the message passing may include the steps of gathering for each node all neighboring node embeddings or messages, aggregating or pooling all messages via an aggregate function, e.g., a max function and passing all aggregated messages through an update function.

The trained GNN is accordingly obtained by inputting the mapped genomic sequence data and the diagnostic, prognostic and/or predictive conclusions.

In a last step of the method according to the present invention a diagnostic, prognostic and/or predictive conclusion output with respect to said disease or medical condition is derived. The trained GNN is thus used for diagnostic, prognostic and/or predictive purposes in the context of a target genomic sequence, e.g., derived from a subject or patient.

The trained GNN thus produces an output for inputted genomic sequence data in the context of the learned task, allowing to derive a diagnostic, prognostic and/or predictive conclusion with respect to the disease or medical condition originally associated with the training data of the cohort of diseased subjects in the context of the genomic sequence data of the target subject. In other words, the trained GNN produces an output which links a diagnosis, prognosis and/or therapeutic instruction to the inputted genomic sequence data. The output may, for example, be provided as report or alert.

In further specific embodiments, additionally a target subject's data concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome, is obtained and inputted into the trained graph neural network in encoded form. The encoding of this additional information is further concatenated with the embeddings of all genes to yield a layer which is finally inputted into a multilayer perceptron (MLP) producing a prediction label as outcome.

Also envisaged are data on the hospital where the patient was examined, or the laboratory where the analysis was performed, health records including a history of earlier diseases or previous diagnoses. The data may be inputted into the GNN and advantageously be reflected by similar data on age, sex, race etc. introduced in the training phase.

In a further specific embodiment, the training of the trained graph neural network as mentioned herein additionally includes the inputting of data of a cohort of subjects affected by a disease or medical condition concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome. Such molecular changes may include changes of a subject's or a cohort of subjects' transcriptome, metabolome, proteome, glycome or lipidome in comparison to a control or reference transcriptome, metabolome, proteome, glycome or lipidome, e.g., obtained from a healthy subject. Importantly, the present invention envisages that merely data may be used which are available for the specific patient cohort analyzed. However, additional information, e.g., from literature databases, such as PubMed or the like, can be employed to select a subset of data, e.g., metabolites, that have already been associated with the disease.

Also envisaged are data on the hospital where the patient was examined, or the laboratory where the analysis was performed, health records including a history of earlier diseases or previous diagnoses. The data may be provided for the entire cohort and introduced into the GNN as additional vectors which are added in encoded form to the network (see also Fig. 1, reference signs (9) and (10)). The encoding of this additional information is further concatenated with the embeddings of all genes to yield a layer (12) which is finally inputted into a multilayer perceptron (MLP) producing a prediction label as outcome.

In a preferred embodiment the diagnostic and/or therapeutic conclusion output comprises a disease subtype classification. For example, the trained GNN may provide a result, which allows to classify the disease according to its subtype. Furthermore, the conclusion output may comprise a prognostic trend assessment. The output may, for example, be based on a previous diagnosis which can, according to certain embodiments, be modified by a prognostic trend definition. Likewise, a treatment response prediction may be provided as outcome. Accordingly, the target subject may receive a statement on the feasibility of certain treatment procedures.

The present invention further envisages in a preferred embodiment that the output is provided in the form of a metric score within a clinical decision scale. The term "metric score" as used herein refers to a graduation scheme which is a part of the GNN's prediction. This graduation scheme is provided as metric classification embedded within a clinical or therapeutic decision scale. For example, the severity of a disease may be classified on a scale from 1 to 10, e.g., 1 being very mildly severe, whereas 10 is strongly severe. This diagnosis may subsequently lead to a corresponding therapeutic decision. Alternatively, the output may be a value for the likelihood of, for example, a 6-month survival, e.g., after mutations causing cancer were detected. In a further example, the output may be a prediction of the time until recurrence of the disease etc. Likewise, the predictive output may be provided as metric score, e.g., a certain dosage may be provided in a metric scale from 1 to 10 times a normalized dose 1, as known to the skilled person, or a radiation therapy may be suggested with a metric graduation from strength 1 to 10 based on a normalized standard strength 5 as known to the skilled person. The calculation may be performed via the form of the prediction MLP, e.g., the number of output neurons and type of activation function, and the loss function. An envisaged example of a binary classification comprises a MLP with an output neuron and a sigmoid activation function and binary cross-entropy loss.

In a further specific step, the diagnostic, prognostic, or predictive output may additionally be finetuned or modified by associating a measure of uncertainty to the metric score as described herein. The term "measure of uncertainty" as used herein refers to a statistical factor which reflects the accuracy of the prediction of the network by considering the similarity between the current molecular situation of a target subject vis-à-vis the molecular situation of the cohort of subjects yielding the training data set. The statistical factor may, for example, be derived from a measurement of the similarity between the latent representation of the target subject's genomic data and the latent representations of the patient cohorts' genomic data. In a further embodiment the statistical factor may be derived from the measurement of patient similarity, e.g., with respect to age, sex, race, health records etc. A measurement of similarity may, for example, be performed with a taxicab or Manhattan geometry, wherein the distance between two points is measured as sum of absolute differences of Cartesian coordinates. For example, in case a target subject's genomic sequence data yields a mutational pattern, which is reflected by mutational data derivable from subjects of the training cohort, more preferably by data of subjects which have been treated successfully, the likelihood of a positive therapeutic response of the target subject is high. In consequence the uncertainty would be low.

In further specific embodiments the trained GNN as defined herein above may be combined with another neural network architecture. Such other neural networks may be associated as submodules responsible for a subgroup of activities. For example, the additional neural networks may explicitly model interactions between genes, or may determine protein-protein interactions, or be based on supervised learning approaches.

In one embodiment the present invention further relates to a computer-implemented method for the analysis of genomic sequence data comprising the steps: (a) obtaining data from three-dimensional protein structures; (b) building a graph neural network architecture from the protein structures of step (a); (c) obtaining genomic sequence data from a cohort of subjects affected by a disease or medical condition; (d) mapping the genomic sequence data obtained in step (c) on the protein structures obtained in step (a); (e) training the graph neural network with the mapped genomic sequence data of step (d) and diagnostic, prognostic and/or predictive conclusions in the context of the disease or medical condition corresponding to the genomic sequence data obtained in step (c), thereby obtaining a trained graph neural network; (f) obtaining a target subject's genomic sequence data; (g) mapping the genomic sequence data obtained in step (f) on the protein structures obtained in step (a); (h) inputting the mapped genomic sequence data of step (g) into the trained graph neural network obtained in step (e); and (i) deriving a diagnostic, prognostic and/or predictive conclusion output with respect to said disease or medical condition. The activities, terms and steps of this method correspond to those defined herein above. The method further envisages all specific embodiments as detailed herein above.

In another embodiment the present invention relates to a computer-implemented method for providing a trained graph neural network for analysis of genomic sequence data comprising the steps: (a) obtaining data from three-dimensional protein structures; (b) building a graph neural network architecture from the protein structures of step (a); (c) obtaining genomic sequence data from a cohort of subjects affected by a disease or medical condition; (d) mapping the genomic sequence data obtained in step (c) on the protein structures obtained in step (a); (e) training the graph neural network with the mapped genomic sequence data of step (d) and diagnostic, prognostic and/or predictive conclusions in the context of the disease or medical condition corresponding to the genomic sequence data obtained in step (c), thereby obtaining a trained graph neural network. The method further envisages all specific embodiments concerning the generation and training of the GNN as detailed herein above. In preferred embodiments, the trained GNN generated in this method may be used for the computer-implemented method for the analysis of genomic sequence data as defined herein above.

The computer-implemented method as described herein may be implemented on any suitable storage or computer platform, e.g., be cloud-based, internet-based, intra-net based or present on local computer or cellphones etc.

In another aspect the present invention relates to a data processing device comprising means for carrying out the computer-implemented method as defined above. The device comprises means for carrying out any one or more steps of the computer-implemented method of the present invention as mentioned herein above. Accordingly, any of the computer-implemented methods described herein may be totally or partially performed with a computer system including one or more processor(s), which can be configured to perform the steps. Accordingly, some of the present embodiments are directed to computer systems configured to perform the steps of any of the computer-implemented methods described herein, potentially with different components performing respective steps or a respective group of steps. Corresponding steps of methods may further be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

Also envisaged is a computer program comprising instructions which, when the program is executed by a computer, cause the computer to carry out any of the computer implemented methods of the invention as defined herein or any one or more computerizable steps of the methods of the present invention as mentioned herein.

Also envisaged is the provision of a computer-readable storage medium comprising a computer program product as defined above. The computer-readable storage medium may be connected to a server element, or be present in a cloud structure, or be connected via internet or intranet to one or more database structures, or client databases etc.

Any of the software components or computer programs or functions described herein may be implemented as software code to be executed by a processor using any suitable computer language such as, for example, Java, Python, JavaScript, VB.Net, C++, C#, C, Swift, Rust, Objective-C, Ruby, PHP, or Perl using, for example, conventional or object-oriented techniques. The software code may be stored as a series of instructions or commands on a computer readable medium for storage and/or transmission, suitable media include random access memory (RAM), a read only memory (ROM), a magnetic medium such as a hard-drive, or an optical medium such as a compact disk (CD) or DVD (digital versatile disk), flash memory, and the like. The computer readable medium may be any combination of such storage or transmission devices. Such programs may also be encoded and transmitted using carrier signals adapted for transmission via wired, optical, and/or wireless networks conforming to a variety of protocols, including the Internet. As such, a computer readable medium according to the present invention may be created using a data signal encoded with such programs. Computer readable media encoded with the program code may be packaged with a compatible device or provided separately from other devices (e.g., via internet download). Any such computer readable medium may reside on or within a single computer program product (e.g., a hard drive, a CD, or an entire computer system), and may be present on or within different computer program products within a system or network. A computer system may include a monitor, printer, or other suitable display for providing any of the results mentioned herein to a user.

In a preferred, specific embodiment the present invention relates to the training and evaluation of a model to predict one-year survival of adenocarcinoma patients from mutation profiles and clinical data. This embodiment comprises the following steps:
(1) Obtaining a somatic mutation profile, e.g., whole-exome sequencing data, and clinical data, e.g., age, sex, pack years for smokers, histological stage, including survival data, e.g., time to overall survival, of lung adenocarcinoma patients. Preferably the lung adenocarcinoma cohort of the TOGA PanCancer Atlas from cBioPortal may be used.
(2) Predicting the functional consequence of single mutations, e.g., by using the Variant Effect Predictor (VEP) .
(3) Selecting genes which are annotated in the COSMIC Cancer Gene Census and carry one or more non-synonymous mutations, e.g., mutations classified as moderate or high functional impact mutations by VEP.
(4) In addition, selecting significantly mutated genes with high prevalence, e.g., TP53, KRAS, KEAP1, STK11, EGFR, NF1, BRAF, or SETD2.
(5) Selecting, for each gene, a corresponding 3D protein structure, e.g., an experimentally determined 3D structure from the Protein Data Bank archive or a Computed Structure Model (CSM).
(6) Calculating, for all proteins, a pairwise Euclidean distance between the Cbeta-atoms of amino acids and applying a threshold (e.g., 7 Å) to obtain protein contact maps.
(7) Using the contact maps as adjacency matrices to build one graph per protein structure.
(8) Mapping mutations to corresponding amino acids and 1-hot encoding mutations using the following categories: absence of a non-synonymous mutation, presence of a moderate impact mutation, or presence of a high impact mutation.
(9) Encoding the clinical data.
(10) Splitting the dataset randomly into training, validation, and test sets while stratifying with respect to one-year survival.
(11) Training the model on the training set, using the validation set for early stopping and model selection, and evaluate the model on the test set.
(12) Iterating steps 10 and 11 to obtain an ensemble of models and calculating the evaluation metric's (e.g., concordance index) mean and variance, as a measure of uncertainty.

In a further preferred, specific embodiment the present invention relates to the application of a pretrained model to predict one-year survival of adenocarcinoma patients from mutation profiles and clinical data. This embodiment comprises the following steps:
1. Obtaining a pretrained model.
2. Obtaining a somatic mutation profile and clinical data of a lung adenocarcinoma patient.
3. Selecting somatic mutation profiles of genes that are modeled in the pretrained model.
4. Predicting the functional consequence of single mutations, e.g., using the Variant Effect Predictor (VEP) .
5. Mapping mutations to corresponding amino acids in the pretrained model and 1-hot encode mutations using the following categories: absence of a non-synonymous mutation, presence of a moderate impact mutation, or presence of a high impact mutation.
6. Encoding the clinical data.
7. Predicting one-year survival by applying the pretrained model.

The following figure is provided for illustrative purposes. It is thus understood that the figure is not to be construed as limiting. The skilled person in the art will clearly be able to envisage further modifications of the principles laid out herein.

## Claims

1. A computer-implemented method for the analysis of genomic sequence data comprising the steps:
(a) obtaining genomic sequence data;
(b) obtaining data from three-dimensional protein structures;
(c) mapping the genomic sequence data obtained in step (a) on the protein structures obtained in step (b);
(d) inputting the mapped genomic sequence data of step (c) into a trained graph neural network, preferably a graph convolutional network, a graph isomorphism network or a graph attention network
- wherein the architecture of the graph neural network is based on the three-dimensional protein structures,
- wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition; and
(e) deriving a diagnostic, prognostic and/or predictive conclusion output with respect to said disease or medical condition.

2. The computer-implemented method of claim 1, wherein the output is provided in the form of a metric score within a clinical decision scale.

3. The computer-implemented method of claim 2, wherein the method additionally comprises a step of associating a measure of uncertainty to the metric score.

4. The computer-implemented method of any one of claims 1 to 3, wherein the data from three-dimensional protein structures include data for protein-protein interactions or protein-protein complexes.

5. The computer-implemented method of any one of claims 1 to 4, wherein the data from three-dimensional protein structures, protein-protein interactions or complexes are derived from a protein structure database or protein structure prediction database, preferably from PDB or AlphaFold DB.

6. The computer-implemented method of any one of claims 1 to 5, wherein the mapping of the genomic sequence data to the protein structure data incorporates information on a predicted impact of mutations on the protein sequence, structure and function, wherein preferably said information on the impact is provided by a variant effect predictor.

7. The computer-implemented method of any one of claims 1 to 6, wherein the training of the graph neural network additionally includes the inputting of data of a cohort of subjects affected by a disease or medical condition concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome.

8. The computer-implemented method of claim 7, wherein data concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome, is obtained and inputted into the trained graph neural network.

9. The computer-implemented method of any one of claims 1 to 8, wherein the graph neural network comprises nodes and edges, wherein said nodes correspond to the Calpha atom of the amino acid glycine or the Cbeta atom of amino acids other than glycine.

10. The computer-implemented method of claim 9, wherein the edges of the graph neural network link the Cbeta or Calpha atoms of the amino acids that have a Euclidian distance of below a predetermined threshold, preferably a threshold of 6 to 12 Angström, more preferably of 7 Angström.

11. The computer-implemented method of claim 10, wherein the edges of the graph neural network comprise weights which correspond to a function of said Euclidian distance.

12. The computer-implemented method of any one of claims 1 to 11, wherein the genomic sequence data is obtained from a panel sequencing, a whole-exome sequencing or a somatic genomic sequencing, wherein optionally a preselection of genomic sequence data with respect to available protein structures, known mutation locations and/or a disease or medical condition of interest is performed.

13. The computer-implemented method of any one of claims 1 to 12, wherein said diagnostic and/or therapeutic conclusion output comprises a disease subtype classification and/or a prognostic trend assessment and/or a treatment response prediction.

14. A data processing device or system for the analysis of genomic sequence data, comprising means for carrying out the method of any one of claims 1 to 13.

15. A computer program comprising instructions which, when the program is executed by a computer, cause the computer to carry out the method of any one of claims 1 to 13.

## Amended claims

### Amended claims in accordance with Rule 137(2) EPC.

1. A computer-implemented method for the analysis of genomic sequence data comprising the steps:
(a) obtaining genomic sequence data;
(b) obtaining data from three-dimensional protein structures;
(c) mapping the genomic sequence data obtained in step (a) on the protein structures obtained in step (b), wherein the mapping comprises a comparison of the nucleotide sequence via the encoded primary amino acid sequence with the three-dimensional structure of the protein;
(d) inputting the mapped genomic sequence data of step (c) into a trained graph neural network, preferably a graph convolutional network, a graph isomorphism network or a graph attention network
- wherein the architecture of the graph neural network is based on the three-dimensional protein structures,
- wherein the graph neural network is trained based on genomic sequence data from a cohort of subjects affected by a disease or medical condition mapped to the three-dimensional protein structures and corresponding and diagnostic, prognostic and/or predictive conclusions in the context of the disease or medical condition, wherein said mapping comprises an assignment of mutations to the protein sequence incorporating information on a predicted impact of mutations on the protein sequence, structure and function; and wherein the graph neural network comprises nodes and edges, wherein said nodes correspond to the Calpha atom of the amino acid glycine or the Cbeta atom of amino acids other than glycine; and
(e) deriving a diagnostic, prognostic and/or predictive conclusion output with respect to said disease or medical condition in the context of said genomic sequence.

2. The computer-implemented method of claim 1, wherein the output is provided in the form of a metric score within a clinical decision scale, wherein said metric score is a metric classification embedded within a clinical or therapeutic decision scale.

3. The computer-implemented method of claim 2, wherein the method additionally comprises a step of associating a measure of uncertainty to the metric score.

4. The computer-implemented method of any one of claims 1 to 3, wherein the data from three-dimensional protein structures include data for protein-protein interactions or protein-protein complexes.

5. The computer-implemented method of any one of claims 1 to 4, wherein the data from three-dimensional protein structures, protein-protein interactions or complexes are derived from a protein structure database or protein structure prediction database, preferably from PDB or AlphaFold DB.

6. The computer-implemented method of any one of claims 1 to 5, wherein said information on the predicted impact of mutations on the protein sequence is provided by a variant effect predictor.

7. The computer-implemented method of any one of claims 1 to 6, wherein the training of the graph neural network additionally includes the inputting of data of a cohort of subjects affected by a disease or medical condition concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome.

8. The computer-implemented method of claim 7, wherein data concerning one or more of: age, sex, race, characteristics of disease phenotypes, histologic characteristics, stage of development of a disease, histologic subtypes, detectable molecular changes, preferably on the level of the transcriptome, metabolome, proteome, glycome or lipidome, is obtained and inputted into the trained graph neural network.

9. The computer-implemented method of any one claims 1 to 8 , wherein the edges of the graph neural network link the Cbeta or Calpha atoms of the amino acids that have a Euclidian distance of below a predetermined threshold, preferably a threshold of 6 to 12 Angstrom, more preferably of 7 Angstrom.

10. The computer-implemented method of claim 9, wherein the edges of the graph neural network comprise weights which correspond to a function of said Euclidian distance.

11. The computer-implemented method of any one of claims 1 to 10, wherein the genomic sequence data is obtained from a panel sequencing, a whole-exome sequencing or a somatic genomic sequencing, wherein optionally a preselection of genomic sequence data with respect to available protein structures, known mutation locations and/or a disease or medical condition of interest is performed.

12. The computer-implemented method of any one of claims 1 or 4 to 11, wherein said diagnostic and/or therapeutic conclusion output comprises a disease subtype classification and/or a prognostic trend assessment and/or a treatment response prediction.

13. A data processing device or system for the analysis of genomic sequence data, comprising means for carrying out the method of any one of claims 1 to 12.

14. A computer program comprising instructions which, when the program is executed by a computer, cause the computer to carry out the method of any one of claims 1 to 12.
